# EUROPÄISCHE PATENTSCHRIFT

(11) **EP 3 346 902 B1**
(45) Veröffentlichungstag und Bekanntmachung des Hinweises auf die Patenterteilung: **07.06.2023**
(21) Anmeldenummer: 16775080.1
(22) Anmeldetag: 07.09.2016
(51) Int. Cl.: A61B 3/00, A61B 3/028, A61B 3/032, A61B 3/04

(54) **SYSTEM ZUR PRÜFUNG DER SEHLEISTUNG UND ZUM ERKENNEN UND KORRIGIEREN VON SEHFEHLERN**
SYSTEM FOR TESTING EYESIGHT AND DETECTING AND CORRECTING VISUAL DEFECTS
SYSTÈME POUR CONTRÔLER LA VISION ET POUR DÉTECTER ET CORRIGER DES TROUBLES DE LA VISION

(30) Priorität: 09.09.2015 DE 102015115202
(43) Veröffentlichungstag der Anmeldung: 18.07.2018
(73) Patentinhaber: Optik Hecht E.K. Inhaber: Gerhard Hecht, 88316 Isny (DE)
(72) Erfinder: HECHT, Gerhard, 88316 Isny (DE)
(74) Vertreter: Keller Schneider Patentanwaltsgesellschaft mbH
(86) Internationale Anmeldenummer: PCT/EP2016/071098
(87) Internationale Veröffentlichungsnummer: WO 2017/042223

(56) Entgegenhaltungen:
- WO-A1-2014/064719
- WO-A1-2014/112626
- DE-A1-102014 223 442
- US-A1- 2011 027 766

## Beschreibung

Die Erfindung betrifft ein System zur Prüfung der Sehleistung und zum Erkennung und Korrigieren von Sehfehlern gemäß dem Oberbegriff des Anspruchs 1. Die Erfindung betrifft auch eine vorteilhafte Verwendung des Systems gemäß dem Patentanspruch 9.

Aus der DE 10 2014 223 442 A1 ist ein Verfahren bekannt, bei dem eine Sehschärfeprüfung und/oder die Prüfung einer Refraktion einer Testperson mit Hilfe eines als 3D-Monitor ausgebildeten Bildausgabegeräts erfolgt. Auf dem 3D-Monitor werden gesteuert durch einen Rechner in einer Sehprobentafel Optotypen so dargestellt, dass sie von den Augen monokular im binokularen 3D-Umfeld wahrgenommen werden.

Aus der WO 2014/112626 A1 und der US 2015/351625 A1 sind ein Prismenvorgabewert-Erfassungssystem, ein Erfassungsverfahren, eine Erfassungsvorrichtung und ein Programm zum Korrigieren einer Fixationsdisparität bekannt.

Ferner ist aus der US 2011/027766 A1 ein System und ein Verfahren zum Testen und/oder Trainieren einer Vielzahl von visuellen Fähigkeiten und Fertigkeiten einer Person und einer effizienten Zeit, und zwar unter Verwendung kompakter, mehrfach verwendbarer Instrumente bekannt.

Außerdem ist aus der WO 2014/064719 A1 ein Netzwerk von Vorrichtungen zur Durchführung optischer/optometrischer/ophthalmologischer Tests bekannt. Das Netzwerk umfasst mindestens eine Patientenschnittstelle, die zur Anzeige dreidimensionaler und/oder stereoskopischer Bilder ausgelegt ist und mit mindestens einem Anzeigebildschirm mit einer verschachtelten, dreidimensionalen Projektions-/Darstellungsfähigkeit versehen ist. Weiterhin umfasst das Netzwerk mindestens eine Bedienerschnittstelle, die zur Anwendung von Steuerungs- und Verwaltungsfunktionen auf die Patientenschnittstelle ausgebildet ist.

Ferner ist aus der DE 10 2014 223 442 A1 ein Verfahren zur Sehzeichendarstellung über ein Bildausgabegerät für eine Sehschärfenprüfung und die Bestimmung einer Refraktion bekannt.

Aufgabe der Erfindung ist die Bereitstellung eines Systems mit einer verbesserten Erkennung, Auswertung und Korrektur von Sehfehlern oder Sehschwächen der menschlichen Augen. Ferner sollen vorteilhafte Verwendungen dieses Systems geschaffen werden.

Diese Aufgabe wird durch die Merkmale des Anspruchs 1 gelöst. Vorteilhafte Ausgestaltungen der Erfindung sind in den Unteransprüchen angegeben. Die vorteilhafte Verwendung des erfindungsgemäßen Systems ist in dem Patentanspruch 9 angegeben.

Die erfinderische Lösung gemäß des Anspruchs 1 sieht insbesondere vor, dass das System wenigstens eine erste Bildanzeigevorrichtung für den Fernbereich und wenigstens eine zweite Bildanzeigevorrichtung für den Nahbereich aufweist, deren Abstand zu den Augen der Testperson unterschiedlich ist, dass weiterhin durch den Rechner mittels einer auf diesem ausführbaren Software Optotypen unterschiedlicher Art, Größe, Farbe, Kontrast und/oder Helligkeit auf den Prüftafeln und unterschiedliche dreidimensionale Hintergründe auf den Bildanzeigevorrichtungen erzeugbar sind, und dass schließlich vor den Augen der Testperson wenigstens eine binokulare Korrekturvorrichtung angeordnet ist, an der unterschiedliche Linsen und/oder Prismen und/oder 3D-Trennfilter aktivierbar sind. Insbesondere die dreidimensionalen Hintergründe sind auch in Form von bewegten Bildern darstellbar. Die Hintergründe sind von ihren Motiven, den verwendeten Farben und der Intensität und Geschwindigkeit der Darstellung auf die Art und die Befindlichkeit der Testperson anpassbar. Dies ist insbesondere bei Kindern als Testperson von sehr großem Vorteil, um deren Aufmerksamkeit für die Dauer der Prüfung zu wecken und zu erhalten. Die optische Führung der Augen der Testperson kann bevorzugt optisch durch einen eingeblendeten auffälligen Lichtpunkt oder ein eingeblendetes Symbol oder durch eine größere Helligkeit des jeweils zu fokussierenden Objekts durch den Rechner auf der jeweiligen Bildanzeigevorrichtung erzeugt werden.

Wenn in dieser Anmeldung der Begriff "3D-Trennfilter" verwendet wird, so sind davon alle Arten von Bildtrennungen für stereoskopisches Sehen umfasst. Wenn in den Patentansprüchen oder im Ausführungsbeispiel von einem "Polarisationsfilter" gesprochen wird, so sind damit stets auch alle anderen Arten von 3D-Trennfiltern mit umfasst.

Wenn in dieser Anmeldung der Begriff " Linsen" verwendet wird, sind davon alle Arten von optischen Linsen umfasst, darunter auch Asphärische Linsen, Gradientenlinsen oder Bifokalgläser aus Glas oder Kunststoff.

Bevorzugt ist vorgesehen, dass die Optotypen jeweils eines Optotypen-Bereichs durch einen an der Korrekturvorrichtung angeordneten Trennfilter nur für eines der Augen erkennbar sind. Generell ist jedes 3D-System zur Darstellung des Hintergrundbildes und der Optotypentafeln geeignet, darunter auch einfache 3D-Trennfilter in Form von Komplementärfarben-Scheiben ("Anaglyphenbrille"), alle Arten von Polarisationsfiltern (lineare und zirkulare) und/oder Interferenzfilter. Bei einer Verwendung eines Monitors als Bildanzeigevorrichtung kommen entsprechend Polarisationsmonitore (passive 3D-Technik mit zirkular polarisiertem Licht), Shuttermonitore (mit schnell abwechselnder Anzeige des rechten und des linken Bildes und aktiver Synchronisation in der Korrekturvorrichtung) oder Spiegelboxen ("Cobox", zwei normale Monitore, die unter Verwendung eines halbdurchlässigen Spiegels zu einem 3D-Sichtgerät angeordnet werden) in Betracht.

Bei einer Verwendung eines Projektors und einer Leinwand als Bildanzeigevorrichtung kommen entsprechend Polarisationsprojektoren und Polarisationsfilter an der Korrekturvorrichtung, Shutter-Projektoren (eventuell in Verbindung mit einer zusätzlichen Polarisation) und Shutterbrille an der Korrekturvorrichtung oder Interferenzfiltertechnik (lnfitec^{®}-Verfahren) mit entsprechenden Interferenzgläsern an der Korrekturvorrichtung in Betracht.

Erfindungsgemäß sind die Abstände und/oder die Positionen der Bildanzeigevorrichtungen relativ zu den Augen der Testperson einstellbar.

Erfindungsgemäß ist vorgesehen, dass mittels der Software die auf der ersten Bildanzeigevorrichtung dargestellten Optotypen und die die auf der zweiten Bildanzeigevorrichtung dargestellten Optotypen wechselweise ein- und ausblendbar oder in ihrer Darstellungsintensität bezüglich Art, Größe, Farbe, Kontrast und/oder Helligkeit wechselweise veränderbar sind. Dadurch können besonders vorteilhaft während der Prüfung insbesondere solche lokalen Netzhaut-Bereiche in den Augen der Testperson erkannt und stimuliert werden, in denen lokal begrenzte Ausfälle von Netzhautarealen, ein spezifisches Sehdefizit oder Wahrnehmungsstörungen vorliegen (zum Beispiel die Ermittlung der Lage des "blinden Flecks" oder beeinträchtigter lokaler Netzhautareale des rechten und/oder des linken Auges).

Erfindungsgemäß ist vorgesehen, dass der Rechner mit der Korrekturvorrichtung verbunden ist und die Software entsprechend einer Rückmeldung der Testperson unterschiedliche Linsen und/oder Prismen an der Korrekturvorrichtung aktiviert.

Erfindungsgemäß ist ferner vorgesehen, dass der Rechner mit wenigstens einer Eingabevorrichtung verbunden ist, mittels der die Testperson oder ein Bediener des Systems aufgrund der Rückmeldungen der Testperson Signale an den Rechner sendet, mittels derer die Software die weitere Erzeugung von Optotypen bezüglich deren Art, Größe, Farbe, Kontrast und/oder Helligkeit und/oder die Aktivierung von Linsen und/oder Prismen an der Korrekturvorrichtung und/oder die Aktivierung von 3D-Trennfiltern auf den Bildanzeigevorrichtungen ansteuert. Bei einem teilautomatischen System erzeugt die Software aufgrund der Rückmeldungen Vorschläge für den weiteren Ablauf des Prüfprogramms und die Aktivierung von Linsen und/oder Prismen an der Korrekturvorrichtung, die von einem Bediener des Systems manuell ausführbar oder aufgrund eigener Erkenntnisse korrigierbar sind.

Es ist weiterhin vorteilhaft, wenn die Optotypen-Bereiche an der zweiten Bildanzeigevorrichtung für den Nahbereich kleiner sind als die Optotypen-Bereiche an der ersten Bildanzeigevorrichtung für den Fernbereich. Dadurch bleibt der Objektwinkel des dargestellten Gegenstands bei einem Wechsel zwischen Fern- und Nahbereich zur Prüfung der Akkomodationsgeschwindigkeit und der Akkomodationsabstimmung zwischen rechtem und linkem Auge vorteilhafterweise im Wesentlichen gleich und damit bleibt auch die Größe des Netzhautbildes des Gegenstandes im Nahbereich in etwa so groß wie der des Gegenstandes im Fernbereich, so dass Auge und Gehirn nicht gleichzeitig noch zu einer zusätzlichen Anpassungsleistung gezwungen werden. Für eine derartige Prüfung im Nahbereich ist insbesondere eine Duan'sche Strichfigur sehr gut geeignet, da diese von Netzhautbildgrößen unabhängig ist.

Gemäß einer weiteren vorteilhaften Weiterbildung des erfindungsgemäßen Systems ist vorgesehen, dass auf wenigstens einer der Bildanzeigevorrichtungen wenigstens ein Bildelement und/der ein Optotypen-Bereich und/oder der Hintergrund stereoskopisch bezüglich der Bildebene in der Tiefe verschiebbar oder in seiner Bildtiefenausdehnung veränderbar ist.

Eine weitere vorteilhafte Ausgestaltung sieht vor, dass basierend auf den Ergebnissen der Prüfung der Augen der Testperson ein individuelles Trainingsprogramm zur Verbesserung der Sehleistung der Testperson auf einem Datenträger gespeichert wird.

Gemäß einer ersten vorteilhaften Verwendung eines erfindungsgemäßen Systems ist vorgesehen, dass die in der Korrekturvorrichtung zur Herstellung eines optimalen Sehergebnisses der Testperson im Nah- und Fernbereich verwendete Konstellation von Linsen und/oder Prismen als Grundlage für die Erstellung einer individuell angepassten Sehhilfe verwendet wird.

Gemäß einer zweiten vorteilhaften Verwendung eines erfindungsgemäßen Systems ist vorgesehen, dass die in der Korrekturvorrichtung zur Herstellung eines optimalen Sehergebnissen der Testperson im Nah- und Fernbereich verwendete Konstellation von Linsen und/oder Prismen sowie die bei der Prüfung erfolgte Ermittlung bestimmter Netzhautbereiche der beiden Augen, in denen gezielte Reize in Form von Hintergründen und/oder Optotypen spezieller Art, Größe, Farbe, Kontrast und/oder Helligkeit eine verbesserte Wahrnehmung bewirken, als Grundlage für die Erstellung eines individuell angepassten, jederzeit reproduzierbaren Trainingsprogramms zur Verringerung oder Behebung eines individuellen visuellen oder wahrnehmungsbedingten Sehdefizits der Testperson verwendet wird.

Nachfolgend wird ein Ausführungsbeispiel einer erfindungsgemäßen Vorrichtung unter Bezugnahme auf die Zeichnung näher erläutert. Es zeigt:

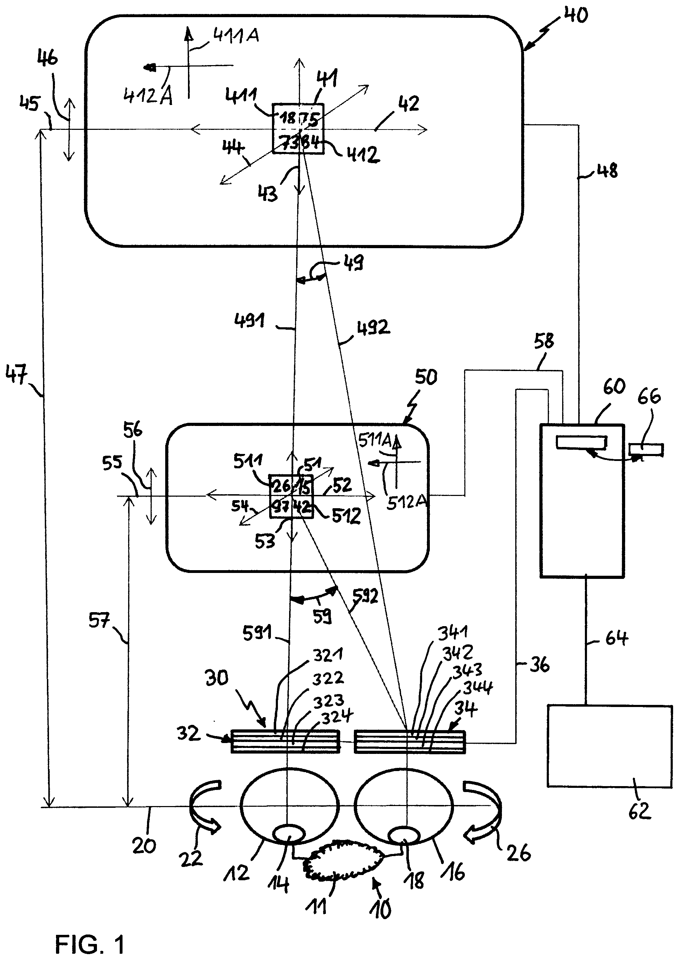

Fig. 1 eine schematische Ansicht eines erfindungsgemäßen Systems.

Das in Figur 1 dargestellte System umfasst eine Testperson 10 mit einem Gehirn 11, einem linken Auge 12 und einem rechten Auge 16, eine Korrekturvorrichtung 30, eine erste Bildanzeigevorrichtung 40 für den Fernbereich, eine zweite Bildanzeigevorrichtung 50 bei den Nahbereich, einen Rechner 60 und eine Eingabevorrichtung 62.

Der Netzhautbereich des linken Auges ist mit 14 und der Netzhautbereich des rechten Auges mit 18 bezeichnet. Die beiden Augen 12 bzw. 16 definieren eine mittlere Sehebene 20 der Testperson 10. Die Pfeile 22 bzw. 26 deuten die mögliche Verrollung der beiden Augen 12 bzw. 16 an, das heißt deren durch die Augenmuskulatur erfolgende Verdrehung um die Sehachse.

Vor den beiden Augen 12 bzw. 16 der Testperson 10 ist die Korrekturvorrichtung 30 angeordnet, die sich aus einem Korrekturbereich 32 für das linke Auge 12 und einem Korrekturbereich 34 für das rechte Auge 16 zusammensetzt. Die Korrekturvorrichtung 30 kann optional auch als brillenähnliche Vorrichtung am Kopf der Testperson 10 angeordnet sein. Im Korrekturbereich 32 sind ein Polarisationsfilter 321 und mehrere einzeln oder in Gruppen aktivierbare Linsen und/oder Prismen 322, 323, 324 zusammengefasst. Dabei ist die Zahl von drei dargestellten Linsen oder Prismen nur exemplarisch gewählt; sie kann in der Realität deutlich höher sein. Analog dazu weist der Korrekturbereich 34 für das rechte Auge 16 einen Polarisationsfilter 341 und mehrere Linsen und/oder Prismen 342, 343, 344 auf, die ebenfalls einzeln oder in Gruppen, synchron oder abweichend von den Linsen und/oder Prismen 322, 323, 324 des Korrekturbereichs 32 aktivierbar sind.

Die Korrekturvorrichtung 30 ist mit dem Rechner 60 über eine Datenleitung 36 verbunden. Sofern die Korrekturvorrichtung 30 als brillenähnliche Vorrichtung am Kopf der Testperson 10 angeordnet ist, kann die Datenleitung 36 auch durch eine drahtlose Datenübertragungsstrecke (mittels Funk, Infrarot, Bluetooth^{®} oder ähnlichem) ersetzt sein. Über die Datenleitung 36 erhält der Rechner 60 Informationen über den aktuellen Status der verwendeten 3D-Trennfilter 321, 341 und/oder Linsen und/oder Prismen 322, 323, 324, 342, 343, 344. Über die Datenleitung 36 erfolgt bei einer erfindungsgemäßen vollautomatischen Korrektureinrichtung 30 auch die Ansteuerung der in dieser enthaltenen, nicht im einzelnen dargestellten Stellmotore für die Aktivierung oder Deaktivierung der einzelnen 3D-Trennfilter 321, 341 und/oder Linsen und/oder Prismen 322, 323, 324, 342, 343, 344.

Die Aktivierung der 3D-Trennfilter und/oder der Linsen und/oder Prismen 322, 323, 324, 342, 343, 344 erfolgt in einem erfindungsgemäßen vollautomatisierten System ähnlich einem Phoropter durch Stellmotore, die vom Rechner 60 angesteuert werden. Dabei werden einzelne oder auch mehrere Linsen und/oder Prismen 322, 323, 324, 342, 343, 344 entsprechend eines auf dem Rechner 60 ablaufenden Prüfprogramms in den Sichtbereich der Augen 12 und/oder 16 hinein geschwenkt oder aus diesem herausgeschwenkt und/oder im Sichtbereich der Augen 12,16 verschoben und/oder gedreht. Mittels der Eingabevorrichtung 62 kann der Bediener hierbei beispielsweise mittels eines Joystick-ähnlichen Bedienorgans selbst die nötigen Korrekturen an der Korrektureinrichtung 30 für ein optimales Sehergebnis vornehmen.

In einem teilautomatisierten System stellt ein Bediener von Hand die jeweils für den nächsten Prüfschritt des Prüfprogramms zu verwendenden 3D-Trennfilter 321, 341 und/oder Linsen und/oder Prismen 322, 323, 324, 342, 343, 344 gemäß den vom Prüfprogramm durch vorausgegangene Rückmeldung der Testperson 10 und eine entsprechende Eingabe an der mit dem Rechner 60 über eine Datenleitung 64 verbundenen Eingabevorrichtung 62 erarbeiteten Vorschlägen in den Korrekturbereichen 32 und 34 der Korrekturvorrichtung 30 ein.

Die erste Bildanzeigevorrichtung 40 für den Fernbereich ist im gezeigten Ausführungsbeispiel als 3D-Monitor 40 ausgebildet. Dieser befindet sich bevorzugt in einem Abstand 47 von etwa 4 m oder mehr von der mittleren Sehebene 20 der Testperson 10. Der Abstand 47 entspricht somit etwa 0,25 Dioptrien. Die erste Bildanzeigevorrichtung 40 weist bevorzugt eine Bildschirmgröße von etwa 60 Zoll auf und verfügt bevorzugt über eine Full-HD (1920 x 1080 Pixel) oder besser sogar über eine Ultra HD- 4k-Bildschirmauflösung (mit 3840 x 2160 Pixeln bzw. mit 4096 x 2160 Pixeln beim Cinema 4k-Format).

Eine Horizontalachse 42 und eine Vertikalachse 43 definieren mögliche Bewegungsrichtungen angezeigter Objekte in der Ebene des 3D-Monitors 40. Die Bildtiefenachse 44 definiert stereoskopische Bewegungen angezeigter Objekte aus der Anzeigefläche bzw. Bildebene 45 des 3-D-Monitors 40 heraus oder in dessen Tiefe hinein.

Auf der Anzeigefläche des 3D-Monitors 40 ist außer stehenden oder bewegten Hintergrundbildern wenigstens eine Prüftafel 41 darstellbar. Auf der Prüftafel 41 befindet sich ein erster Optotypen-Bereich 411 mit Optotypen verschiedener Art, Größe, Farbe und Intensität, die das rechte Auge 16 der Testperson 10 ansprechen. Die selektive Wahrnehmung der Optotypen des ersten Optotypen-Bereichs 411 durch das rechte Auge 16 erfolgt dabei im Ausführungsbeispiel durch eine zirkulare Polarisation des Bildes im ersten Optotypen-Bereich 411 in Verbindung mit einem entsprechenden Polarisationsfilter 341 an der Korrektureinrichtung 30.

Unterhalb des ersten Optotypen-Bereichs 411 ist auf der Prüftafel 41 ein zweiter Optotypen-Bereich 412 vorgesehen, dessen Optotypen ebenfalls mit verschiedener Art, Größe, Farbe und Intensität dargestellt werden können und vom linken Auge 12 der Testperson 10 wahrgenommen werden. Die selektive Wahrnehmung der Optotypen des zweiten Optotypen-Bereichs 412 erfolgt im Ausführungsbeispiel ebenfalls durch eine zirkulare Polarisation des Bildes im zweiten Optotypen-Bereich 412 in Verbindung mit einem entsprechenden Polarisationsfilter 321 an der Korrektureinrichtung 30.

Die Prüftafel 41 ist in der Darstellung gemäß Figur 1 zentral auf der Anzeigefläche des 3D-Monitors 40 abgebildet. Die Prüftafel 41 kann insgesamt durch Steuerbefehle des Rechners 60, der mit einer Datenleitung 48 mit dem 3D-Monitor 40 verbunden ist gemäß der Achsen 42, 43 und 44 sowohl in der Bildebene 45 als auch senkrecht dazu in der Bildtiefe verschoben und/oder gedreht werden. Bei Bedarf können auch die beiden Optotypen-Bereiche 411 und 412 separat unabhängig voneinander verschoben und/oder gedreht und/oder zur optischen Führung der Testperson 10 mit unterschiedlicher Helligkeit dargestellt werden. Anstelle der Prüftafel 41 oder ergänzend dazu kann für bestimmte Untersuchungen auch ein aus einem Vertikalbalken 411A und einem Horizontalbalken 412A gebildetes Balkenkreuz auf der Anzeigefläche des 3D-Monitors 40 dargestellt werden. Durch Polarisation ist der Vertikalbalken 411A nur für das rechte Auge 16 und der Horizontalbalken 412A nur für das linke Auge 12 der Testperson 10 wahrnehmbar.

Über eine Vertikalverstellung 46 kann der 3D-Monitor 40 vertikal verstellt werden, um die Horizontalachse 42 in die gewünschte Position zur mittleren Seh-Ebene 20 der Testperson 10 zu bringen. Idealer Weise ist die horizontale Mittelachse des 3D-Monitors 40 auf Augenhöhe der Testperson 10 angeordnet.

Die zweite Bildanzeigevorrichtung 50 für den Nahbereich ist im gezeigten Ausführungsbeispiel ebenfalls als 3D-Monitor 50 ausgebildet. Dieser befindet sich bevorzugt in einem Abstand 57 von etwa 0,3 m bis etwa 1 m von der mittleren Sehebene 20 der Testperson 10. Der Abstand 57 entspricht dabei etwa 1 bis 3 Dioptrien. Die zweite Bildanzeigevorrichtung 50 weist bevorzugt eine Bildschirmgröße von etwa 27 Zoll auf und verfügt bevorzugt über eine Full-HD (1920 x 1080 Pixel) oder besser sogar über eine UHD- 4k-Bildschirmauflösung (mit 3840 x 2160 Pixeln bzw. mit 4096 x 2160 Pixeln beim Cinema 4k-Format).

Eine Horizontalachse 52 und eine Vertikalachse 53 definieren mögliche Bewegungsrichtungen angezeigter Objekte in der Ebene 55 des 3-D-Monitors 40. Die Bildtiefenachse 54 definiert stereoskopische Bewegungen angezeigter Objekte aus der Anzeigefläche bzw. Bildebene 55 des 3-D-Monitors 50 heraus oder in dessen Tiefe hinein.

Auf der Anzeigefläche des 3-D-Monitors 50 ist außer stehenden oder bewegten Hintergrundbildern wenigstens eine Prüftafel 51 darstellbar. Auf der Prüftafel 51 befindet sich ein erster Optotypen-Bereich 511 mit Optotypen verschiedener Art, Größe, Farbe und Intensität, die das rechte Auge 16 der Testperson 10 ansprechen. Die selektive Wahrnehmung der Optotypen des ersten Optotypen-Bereichs 511 durch das rechte Auge 16 erfolgt dabei im Ausführungsbeispiel durch eine zirkulare Polarisation des Bildes im zweiten Optotypen-Bereich 511 in Verbindung mit einem entsprechenden Polarisationsfilter 341 an der Korrektureinrichtung 30.

Unterhalb des ersten Optotypen-Bereichs 511 ist auf der Prüftafel 51 ein zweiter Optotypen-Bereich 512 vorgesehen, dessen Optotypen ebenfalls mit verschiedener Art, Größe, Farbe und Intensität dargestellt werden können, die vom linken Auge 12 der Testperson 10 wahrgenommen werden. Die selektive Wahrnehmung der Optotypen des zweiten Optotypen-Bereichs 512 erfolgt im Ausführungsbeispiel ebenfalls durch eine zirkulare Polarisation des Bildes im zweiten Optotypen-Bereich 512 in Verbindung mit einem entsprechenden Polarisationsfilter 321 an der Korrektureinrichtung 30.

Die Prüftafel 51 ist in der Darstellung gemäß Figur 1 zentral auf der Anzeigefläche des 3D-Monitors 50 abgebildet. Die Prüftafel 51 kann insgesamt durch Steuerbefehle des Rechners 60, der mit einer Datenleitung 58 mit dem 3-D-Monitor 50 verbunden ist, gemäß der Achsen 52, 53 und 54 sowohl in der Bildebene 55 als auch senkrecht dazu in der Bildtiefe verschoben und/oder gedreht werden. Bei Bedarf können auch die beiden Optotypen-Bereiche 511 und 512 separat unabhängig voneinander verschoben und/oder gedreht werden. Anstelle der Prüftafel 51 oder ergänzend dazu kann für bestimmte Untersuchungen auch ein aus einem Vertikalbalken 511A und einem Horizontalbalken 512A gebildetes Balkenkreuz auf der Anzeigefläche des 3D-Monitors 50 dargestellt werden. Durch Polarisation ist der Vertikalbalken 511A nur für das rechte Auge 16 und der Horizontalbalken 512A nur für das linke Auge 12 der Testperson 10 wahrnehmbar.

Über eine Vertikalverstellung 56 kann der 3D-Monitor 50 vertikal verstellt werden, um die Horizontalachse 52 in die gewünschte Position zur mittleren Seh-Ebene 20 der Testperson 10 zu bringen. Idealer Weise ist der 3D-Monitor 50 so angeordnet, dass seine Oberkante vom Auge der Testperson aus betrachtet direkt an die Unterkante des 3D-Monitors 40 anschließt.

Die exemplarisch in den Optotypen-Bereich 411, 412, 511 oder 512 als Ziffern dargestellten Optotypen können selbstverständlich auch durch andere Symbole, wie Buchstaben, Bilder, Linien-Muster, Landoltringe, Snellen-Haken, Random.dot-Bilder oder ähnliches ersetzt werden, wobei die Auswahl sich auch am Alter, am Bildungsstand und den kognitiven und sprachlichen Fähigkeiten der Testperson zur Erfassung und Wiedergabe des Gesehenen orientiert.

Gemäß einer vorteilhaften Weiterbildung des erfindungsgemäßen Systems können die erste Bildanzeigevorrichtung 40 und/oder die zweite Bildanzeigevorrichtung 50 bezüglich der Testperson 10 in ihren Abständen 47 bzw. 57 und/oder in ihrer horizontalen oder vertikalen Position verschiebbar angeordnet sein. Eine relative Verschiebung des Abstandes der ersten und zweiten Bildanzeigevorrichtung 40 bzw. 50 zur Testperson 10 kann auch dadurch erfolgen, dass ein vorzugsweise mit der Korrekturvorrichtung 30 verbundener Sitzplatz der Testperson 10 relativ zu den Bildanzeigevorrichtungen 40 bzw. 50 in seinem Abstand 47 bzw. 57 und optional auch seitlich und in der Höhe verfahrbar ist.

Ein auf der ersten Bildanzeigevorrichtung 40 dargestellter Gegenstand wird vom linken Auge 12 und dem rechten Auge 16 unter einem Objektwinkel 49 wahrgenommen, der von den Bildachsen 491 bzw. 492 begrenzt wird.

Ein auf der zweiten Bildanzeigevorrichtung 50 dargestellter Gegenstand wird vom linken Auge 12 und dem rechten Auge 16 unter einem Objektwinkel 59 wahrgenommen, der von den Bildachsen 591 bzw. 592 begrenzt wird.

Der horizontale Abstand der am stärksten sensitiven Netzhautbereiche 14 des linken Auges 12 und 18 des rechten Auges 16 beträgt durchschnittlich bei einem erwachsenen Menschen etwa 62 bis 74 mm, im Mittel etwa 65 mm und bei Kindern etwa 50 mm. Die Korrekturbereiche 32 und 34 sind durch eine relative horizontale Verschiebbarkeit zueinander auf den entsprechenden Augenabstand anpassbar.

Um die Größe des jeweiligen Netzhautbildes in etwa gleich groß zu halten, ist die Prüftafel 51 für den Nahbereich vorzugsweise kleiner als die Prüftafel 41 für den Fernbereich. Dadurch wird gleichzeitig erreicht, dass die Objektwinkel 49 bzw. 59 annähernd gleich groß sind.

Die Akkommodation des rechten Auges 16 und des linken Auges 12 können mit Hilfe des erfindungsgemäßen Systems sehr gut mit dem Vergenzverhalten abgestimmt werden. Hierzu kann das Stereobild an der zweiten Bildanzeigevorrichtung 50 für den Nahbereich entlang der Achse 54 weiter nach vorne oder nach hinten verschoben werden und dabei gleichzeitig eine Korrektur an den Korrekturbereichen 32 bzw. 34 der Korrekturvorrichtung 30 durch Veränderung der Linsen und/oder Prismen 322, 323, 324, 342, 343, 344 vorgenommen werden.

Durch eine gezielte Veränderung der Art, der Größe, der Farbe, der Helligkeit und des Kontrast der dargestellten Optotypen können spezifische visuelle oder wahrnehmungsbedingte Sehdefizite der Testperson 10 sogar spezifisch für bestimmte lokale Netzhautareale 14 bzw. 18 erkannt werden. Außerdem kann die Position des sogenannten "blinden Flecks" des linken Auges 12 und des rechten Auges 16 exakt bestimmt werden.

Die optische Führung der Augen der Testperson kann bevorzugt optisch durch einen eingeblendeten auffälligen Lichtpunkt oder ein eingeblendetes Symbol oder durch eine größere Helligkeit des jeweils zu fokussierenden Objekts 41; 51; 411, 412; 511, 512 durch den Rechner 60 auf der jeweiligen Bildanzeigevorrichtung 40 bzw. 50 erzeugt werden. Die optische Führung der Testperson 10 kann durch eine akustische Führung durch einen von der Software angesteuerten Lautsprecher oder durch den Bediener unterstützt oder ersetzt werden. Auch bei der Auswahl der Führung der Testperson 10 durch das Testprogramm berücksichtigt das System Alter, Bildungsstand und Wahrnehmungsfähigkeit der Testperson 10, um diese optimal zu unterstützen und ihr Gehirn 11 von zusätzlichen Leistungen während des Testprogramms weitestgehend zu entlasten.

Eine alternierende Veränderung der Intensität der Darstellung auf der Prüftafel 41 für den Fernbereich und auf der Prüftafel 51 für den Nahbereich ermöglicht die Messung der Akkommodationsleistung und Akkommodationsgeschwindigkeit beider Augen 12 bzw. 16 separat oder im Zusammenspiel. Dabei kann zusätzlich eine Verschiebung der Optotypen-Bereiche 411 bzw. 412 und/oder 511 bzw. 512 längs der jeweiligen Bildtiefenachse 44 bzw. 54 weiter nach vorne oder weiter nach hinten erfolgen.

Durch eine Veränderung des Hintergrundbildes bzw. eines Hintergrund-Videos auf der ersten Bildanzeigevorrichtung 40 für den Fernbereich und/oder der zweiten Bildanzeigevorrichtung 50 bei den Nahbereich können die peripheren Bereiche der Netzhautareale 14 bzw. 18 in gewünschter Weise beeinflusst, d.h. stärker stimuliert oder beruhigt werden.

Bei jeder Veränderung des dargestellten Bildes gibt die Testperson 10 eine Rückmeldung über die Qualität des wahrgenommenen Bildes. Entsprechend dieser Rückmeldung, die mittels der Eingabevorrichtung 62 dem Rechner 60 übermittelt wird, werden an den Korrekturbereichen 32 bzw. 34 der Korrekturvorrichtung durch Veränderung der Linsen und/oder Prismen 322, 323, 324, 342, 343, 344 Korrekturen des wahrgenommenen Bildes vorgenommen und entsprechend einer daraufhin erfolgenden Rückmeldung der Testperson 10 vom Prüfprogramm die weiteren Prüfschritte vorgeschlagen bzw. automatisch angesteuert. Vorteilhaft ist insbesondere eine Rückmeldung in Form einer unmittelbaren Eingabe der Testperson 10 in die Eingabevorrichtung 62.

Alternativ oder ergänzend zu den Rückmeldungen der Testperson kann die Korrekturvorrichtung 30 auch mit Sensoren oder Kameras versehen sein, die die durch das jeweilige Bild aktivierten Netzhautareale 14 bzw. 18 erkennt und an den Rechner 60 übermittelt.

Optional sind an der Korrekturvorrichtung 30 auch Sensoren vorgesehen, die Bewegungen des Kopfes der Testperson 10 erkennen und an den Rechner 60 übermitteln. Bei einer Ausbildung der Korrekturvorrichtung 30 in Form einer brillenähnlichen Vorrichtung, die am Kopf der Testperson 10 getragen wird, können diese Sensoren beispielsweise translatorische, rotatorische oder gyroskopische Sensoren sein, die von einer Normallage abweichende Bewegungen, Neigungen und Drehungen des Kopfes der Testperson 10 erkennen.

Das erfindungsgemäße System weist gegenüber allen bisher bekannten Systemen den Vorteil auf, dass das erfindungsgemäße System unter äußerst realitätsnahen Bedingungen die Stärken und Schwächen beider Augen 12 bzw. 16 sowohl einzeln als auch im Zusammenspiel und sowohl statisch als auch dynamisch mit einer bezüglich der Geschwindigkeit einstellbaren wechselnden Veränderung zwischen dem Nahbereich und dem Fernbereich erfasst, wobei aufgrund der Möglichkeit einer sehr genauen Stimulierung einzelner Netzhautareale 14 bzw. 18 auch kleinste, sonst nicht wahrnehmbare Abweichungen registriert werden können..

Das erfindungsgemäße System eignet sich zum einen für eine Verwendung, bei der die in der Korrekturvorrichtung 30 zur Herstellung eines optimalen Sehergebnissen der Testperson 10 im Nah- und Fernbereich verwendete Konstellation von Linsen und/oder Prismen 322, 323, 324, 342, 343, 344 als Grundlage für die Erstellung einer individuell angepassten Sehhilfe verwendet wird. Dabei wird der exakte Status der beim optimalen Sehergebnis verwendeten Konstellation von Linsen und/oder Prismen 322, 323, 324, 342, 343, 344 im Rechner 60 in einem der Testperson 10 zugeordneten Datensatz abgespeichert. Dieser Datensatz kann unmittelbar per Datenfernübertragung an einen Hersteller für die Fertigung einer darauf zugeschnittenen spezifischen Sehhilfe übersendet werden.

Das erfindungsgemäße System eignet sich aber auch hervorragend für eine weitere Verwendung, bei der die vom Prüfprogramm im Verlauf der Ermittlung des optimalen Sehergebnisses erkannten Schwächen des linken Auges 12 und/oder des rechten Auges 16 in Form eines ein auf die jeweilige Testperson 10 zugeschnittenen Trainingsprogramms mittels der auf dem Rechner 60 vorhandenen Software aufgezeichnet und auf einen Datenträger 66 übertragen werden. Der Datenträger 66 kann von der Testperson 10 dann in einer beispielsweise von einem Augenoptiker betriebenen professionellen Sehtrainingseinrichtung oder mit einer bevorzugt ebenfalls auf dem Datenträger 66 gespeicherten vereinfachten Abspielversion der Software auf einem heimischen Rechner in Verbindung mit einem heimischen 3D-Monitor wiederholt als Trainingsprogramm abgespielt werden, um dadurch aufgrund adaptierter schlechter Sehgewohnheiten entstandene Sehdefizite gezielt zu verringern oder gar vollständig zu beheben.

## Patentansprüche

1. System zur Prüfung der Sehleistung und zum Erkennen und Korrigieren von Sehfehlern einer menschlichen Testperson (10) mit zwei Augen (12; 16), mit wenigstens einer zur Darstellung dreidimensionaler Bilder ausgelegten Bildanzeigevorrichtung (40; 50), auf der wenigstens eine Prüftafel (41; 51) mit wenigstens zwei Optotypen-Bereichen (411, 412; 511, 512) darstellbar ist, und mit wenigstens einem Rechner (60) eingerichtet zur Ansteuerung der wenigstens einen Bildanzeigevorrichtung (40; 50),
wobei das System wenigstens eine erste Bildanzeigevorrichtung (40) für den Fernbereich und wenigstens eine zweite Bildanzeigevorrichtung (50) für den Nahbereich aufweist, deren Abstand (47; 57) zu den Augen (12; 16) der Testperson (10) unterschiedlich ist, wobei die Abstände (47; 57) der Bildanzeigevorrichtungen (40; 50) und/oder deren Positionen relativ zu den Augen (12; 16) der Testperson (10) einstellbar sind,
wobei der Rechner (60) mittels einer auf diesem ausführbaren Software eingerichtet ist, Optotypen unterschiedlicher Art, Größe, Farbe, Kontrast und/oder Helligkeit auf den Prüftafeln (41; 51) und unterschiedliche dreidimensionale Hintergründe auf den Bildanzeigevorrichtungen (40; 50) zu erzeugen, und wobei das System wenigstens eine Korrekturvorrichtung (30) umfasst, die vor den Augen (12; 16) der Testperson (10) angeordnet ist, an der unterschiedliche Linsen und/oder Prismen (322, 323, 324; 342, 343, 344) und/oder 3D-Trennfilter (321; 341) aktivierbar sind und wobei der Rechner (60) mit wenigstens einer Eingabevorrichtung (62) verbunden ist, mittels der die Testperson (10) oder ein Bediener des Systems aufgrund der Rückmeldungen der Testperson (10) Signale an den Rechner (60) sendet, mittels derer die Software die weitere Erzeugung von Optotypen bezüglich deren Art, Größe, Farbe, Kontrast und/oder Helligkeit und/oder von Hintergrundbildern oder -Videos auf den Bildanzeigevorrichtungen (40; 50) und/oder die Aktivierung von Linsen und/oder Prismen (322, 323, 324; 342, 343, 344) an der Korrekturvorrichtung (30) und/oder die Aktivierung von 3D-Trennfiltern (321; 341) auf den Bildanzeigevorrichtungen (40; 50) ansteuert, **dadurch gekennzeichnet, dass** die Korrekturvorrichtung (30) als vollautomatische Korrekturvorrichtung (30) mit wenigstens einem Stellmotor ausgebildet ist, wobei der wenigstens eine Stellmotor zur Aktivierung oder Deaktivierung der 3D-Trennfilter (321, 341) und/oder der Linsen und/oder Prismen (322, 323, 324, 342, 343, 344) ausgebildet ist, wobei der Rechner (60) dazu eingerichtet ist, den wenigstens einen Stellmotor anzusteuern.

2. System nach Anspruch 1, **dadurch gekennzeichnet, dass** die Optotypen jeweils eines Optotypen-Bereichs (411, 412; 511, 512) durch einen an der Korrekturvorrichtung (30) angeordneten 3D-Trennfilter (321; 341) nur für eines der Augen (12; 16) erkennbar sind.

3. System nach einem der vorhergehenden Ansprüche, **dadurch gekennzeichnet, dass** mittels der Software die auf der ersten Bildanzeigevorrichtung (40) dargestellten Optotypen und die die auf der zweiten Bildanzeigevorrichtung (50) dargestellten Optotypen wechselweise ein- und ausblendbar oder in ihrer Darstellungsintensität bezüglich Art, Größe, Farbe, Kontrast und/oder Helligkeit wechselweise veränderbar sind.

4. System nach einem der vorhergehenden Ansprüche, **dadurch gekennzeichnet, dass** der Rechner (60) mit der Korrekturvorrichtung (30) verbunden ist und die Software entsprechend einer Rückmeldung der Testperson (10) unterschiedliche Linsen und/oder Prismen (322, 323, 324; 342, 343, 344) an der Korrekturvorrichtung (30) aktiviert.

5. System nach einem der vorhergehenden Ansprüche, **dadurch gekennzeichnet, dass** die Optotypen-Bereiche (511, 512) an der zweiten Bildanzeigevorrichtung (50) für den Nahbereich kleiner sind als die Optotypen-Bereiche (411, 412) an der ersten Bildanzeigevorrichtung (40) für den Fernbereich.

6. System nach einem der vorhergehenden Ansprüche, **dadurch gekennzeichnet, dass** auf wenigstens einer der Bildanzeigevorrichtungen (40; 50) wenigstens ein Bildelement und/oder ein Optotypen-Bereich (411, 412; 511, 512) und/oder der Hintergrund stereoskopisch bezüglich der Bildebene in der Tiefe verschiebbar oder seine Bildtiefenausdehnung bezüglich der Bildebene veränderbar ist.

7. System nach einem der vorhergehenden Ansprüche, **dadurch gekennzeichnet, dass** basierend auf den Ergebnissen der Prüfung der Augen (12; 16) der Testperson (10) ein individuelles Trainingsprogramm zur Verbesserung der Sehleistung der Testperson (10) auf einem Datenträger (66) gespeichert wird.

8. System nach einem der vorhergehenden Ansprüche, **dadurch gekennzeichnet, dass** die Korrekturvorrichtung (30) in Form einer brillenähnlichen Vorrichtung ausgebildet ist, die am Kopf der Testperson (10) angeordnet ist.

9. Verwendung eines Systems nach einem der vorhergehenden Ansprüche, **dadurch gekennzeichnet, dass** die in der Korrekturvorrichtung (30) zur Herstellung eines optimalen Sehergebnissen der Testperson (10) im Nah-und Fernbereich verwendete Konstellation von Linsen und/oder Prismen (322, 323, 324; 342, 343, 344) als Grundlage für die Erstellung einer individuell angepassten Sehhilfe verwendet wird.

## Claims

1. A system for testing eyesight and detecting and correcting visual defects of a human subject (10) with two eyes (12; 16), with at least one image display device (40; 50), which is designed to display three-dimensional images and on which at least one test chart (41; 51) containing at least two optotype zones (411, 412; 511, 512) can be displayed, and with at least one computer (60) adapted for controlling the at least one image display device (40; 50),
wherein the system includes at least one first image display device (40) for the far range and at least one second image display device (50) for the close range whose distances (47; 57) differ relative to the eyes (12; 16) of the subject (10), wherein the distances (47, 57) of the image display devices (40, 50) and/or the positions thereof are adjustable relative to the eyes (12, 16) of the subject (10),
wherein the computer (60) is adapted for generating, by means of software that can be run thereon, optotypes of different types, sizes, colors, contrasts and/or brightness on the test charts (41; 51) and different three-dimensional backgrounds on the image display devices (40; 50), and
wherein the system comprises at least one corrective device (30), which is disposed in front of the eyes (12; 16) of the subject (10), the corrective device (30) having different lenses and/or prisms (322, 323, 324; 342, 343, 344) and/or 3D separating filters (321; 341) that can be activated, and
wherein the computer (60) is connected to at least one input device (62) by means of which the subject (10) or a system's operator sends, due to the return messages by the subject (10), signals to the computer (60) by means of which the software controls the further generation of optotypes with regard to the types, sizes, colors, contrasts and/or brightness thereof and/or of background images or videos on the image display devices (40, 50) and/or the activation of lenses and/or prisms (322, 323, 324, 342, 343, 344) on the corrective device (30) and/or the activation of 3D separating filters (321, 341) on the image display devices (40, 50),
**characterized in that** the corrective device (30) is configured as a fully automated corrective device (30) having at least one actuator, wherein the at least one actuator is configured for activating or deactivating the 3D separating filters (321, 341) and/or lenses and/or prisms (322, 323, 324, 342, 343, 344), wherein the computer (60) is adapted to control the actuator.

2. The system according to claim 1, **characterized in that** the optotypes of each optotype zone (411, 412; 511, 512) are detectable by means of a 3D separating filter (321; 341), which is disposed on the corrective device (30), only for one of the eyes (12; 16).

3. The system according to any one of the previous claims, **characterized in that**, using the software, the optotypes that are visualized on the first image display device (40) and the optotypes that are visualized on the second image display device (50) can alternately be faded in and out, or they are alternately adjustable in terms of the display intensity relative to types, sizes, colors, contrasts and/or brightness.

4. The system according to any one of the previous claims, **characterized in that** the computer (60) is connected to the corrective device (30) and the software activates, according to a return message by the subject (10), different lenses and/or prisms (322, 323, 324; 342, 343, 344) on the corrective device (30).

5. The system according to any one of the previous claims, **characterized in that** the optotype zones (511, 512) on the second image display device (50) for the close range are smaller than the optotype zones (411, 412) on the first image display device (40) for the far range.

6. The system according to any one of the previous claims, **characterized in that**, at least in one of the image display devices (40; 50), at least one image element and/or one optotype zone (411, 412; 511, 512) and/or the background can be stereoscopically shifted in terms of the depth with regard to the image plane, or **in that** the extension of the image depth thereof can be changed relative to the image plane.

7. The system according to any one of the previous claims, **characterized in that**, based on the results of the testing of the eyes (12; 16) of the subject (10), an individual training program for improving the eyesight of the subject (10) is saved on a data carrier (66).

8. The system according to any one of the previous claims, **characterized in that** the corrective device (30) is configured in a form of a device resembling a pair of glasses being arranged on the subject's head.

9. A use of a system according to any one of the previous claims, **characterized in that** the constellation of lenses and/or prisms (322, 323, 324; 342, 343, 344) used in the corrective device (30) for achieving an optimal vision result in the subject (10) in the close range and in the far range serves as a basis for devising an individually adapted vision aid.

## Revendications

1. Système pour tester une performance visuelle et pour détecter et corriger des défauts visuels d'un sujet humain (10) avec deux yeux (12 ; 16), avec au moins un dispositif d'affichage d'images (40 ; 50) conçu pour représenter des images tridimensionnelles, sur lequel au moins un panneau de test (41 ; 51) avec au moins deux zones d'optotypes (411, 412 ; 511, 512) est affichable, et avec au moins un ordinateur (60) conçu pour commander l'au moins un dispositif d'affichage d'images (40 ; 50),
le système présentant au moins un premier dispositif d'affichage d'images (40) pour la zone de loin et au moins un deuxième dispositif d'affichage d'images (50) pour la zone de près, dont la distance (47 ; 57) par rapport aux yeux (12 ; 16) du sujet humain (10) est différente, les distances (47 ; 57) des dispositifs d'affichage d'images (40 ; 50) et/ou leurs positions par rapport aux yeux (12 ; 16) du sujet humain (10) étant réglables,
dans lequel l'ordinateur (60), au moyen d'un logiciel exécutable sur celui-ci, est configuré pour pouvoir générer des optotypes de différents types, tailles, couleurs, contrastes et/ou luminosités sur les panneaux de contrôle (41 ; 51) et différents arrière-plans tridimensionnels sur les dispositifs d'affichage d'images (40 ; 50), et dans lequel le système comprend au moins un dispositif de correction (30) qui est placé devant les yeux (12 ; 16) du sujet humain (10), sur lequel différentes lentilles et/ou prismes (322, 323, 324 ; 342, 343, 344) et/ou filtres de séparation 3D (321 ; 341) sont activables et dans lequel l'ordinateur (60) étant relié à au moins un dispositif d'entrée (62) au moyen duquel le sujet humain (10) ou un opérateur du système envoie des signaux à l'ordinateur (60) sur la base des signaux de retour du sujet humain (10), signaux au moyen desquels le logiciel est en mesure de poursuivre la génération d'optotypes en ce qui concerne leur type, leur taille, leur couleur, leur contraste et/ou leur luminosité et/ou d'images ou de vidéos d'arrière-plan sur les dispositifs d'affichage d'images (40 ; 50) et/ou l'activation de lentilles et/ou de prismes (322, 323, 324 ; 342, 343, 344) sur le dispositif d'affichage d'images (30) et/ou l'activation de filtres de séparation 3D (321 ; 341) sur les dispositifs d'affichage d'images (40 ; 50),
**caractérisé en ce que** le dispositif de correction (30) est conçu comme un dispositif de correction (30) entièrement automatique avec au moins un servomoteur, l'au moins un servomoteur étant conçu pour activer ou désactiver les filtres de séparation 3D (321, 341) et/ou les lentilles et/ou les prismes (322, 323, 324, 342, 343, 344), l'ordinateur (60) étant conçu pour commander l'au moins un servomoteur.

2. Système selon la revendication 1, **caractérisé en ce que** les optotypes de chaque zone d'optotypes (411, 412 ; 511, 512) ne sont reconnaissables que pour l'un des yeux (12 ; 16) grâce à un filtre de séparation 3D (321 ; 341) disposé sur le dispositif de correction (30).

3. Système selon l'une des revendications précédentes, **caractérisé en ce que**, au moyen du logiciel, les optotypes représentés sur le premier dispositif d'affichage d'images (40) et les optotypes représentés sur le deuxième dispositif d'affichage d'images (50) sont configurés pour pouvoir être alternativement affichés et masqués ou leur intensité de représentation modifiée alternativement en ce qui concerne le type, la taille, la couleur, le contraste et/ou la luminosité.

4. Système selon l'une des revendications précédentes, **caractérisé en ce que** l'ordinateur (60) est relié au dispositif de correction (30) et que le logiciel active différentes lentilles et/ou prismes (322, 323, 324 ; 342, 343, 344) sur le dispositif de correction (30) en fonction d'un retour d'information du sujet humain (10).

5. Système selon l'une des revendications précédentes, **caractérisé en ce que** les zones d'optotypes (511, 512) sur le deuxième dispositif d'affichage d'images (50) pour la zone de près sont plus petites que les zones d'optotypes (411, 412) sur le premier dispositif d'affichage d'images (40) pour la zone lointaine.

6. Système selon l'une quelconque des revendications précédentes, **caractérisé en ce que**, sur au moins l'un des dispositifs d'affichage d'images (40 ; 50), au moins un élément d'image et/ou une zone d'optotypes (411, 412 ; 511, 512) et/ou l'arrière-plan sont déplaçables en profondeur de manière stéréoscopique par rapport au plan d'image ou son extension en profondeur d'image est modifiable par rapport au plan d'image.

7. Système selon l'une des revendications précédentes, **caractérisé en ce que**, sur la base des résultats de l'examen des yeux (12 ; 16) du sujet humain (10), un programme d'entraînement individuel destiné à améliorer la performance visuelle du sujet humain (10) est enregistré sur un support de données (66).

8. Système selon l'une des revendications précédentes, **caractérisé en ce que** le dispositif de correction (30) se présente sous la forme d'un dispositif semblable à des lunettes, qui est placé sur la tête du sujet humain (10).

9. Utilisation d'un système selon l'une des revendications précédentes, **caractérisée en ce que** la constellation de lentilles et/ou de prismes (322, 323, 324 ; 342, 343, 344) utilisée dans le dispositif de correction (30) pour produire un résultat visuel optimal du sujet humain (10) dans la zone de vision de près et de loin est utilisée comme base pour la création d'une aide visuelle adaptée individuellement.
